# EUROPEAN PATENT APPLICATION

(11) **EP 3 574 836 A1**
(43) Date of publication of application: **04.12.2019**
(21) Application number: 18175000.1
(22) Date of filing: 30.05.2018
(51) Int. Cl.: A61B 6/03, A61B 6/00, G06T 11/00

(54) **TEMPORALLY GATED THREE-DIMENSIONAL IMAGING**

(71) Applicant: Koninklijke Philips N.V., 5656 AE Eindhoven (NL)
(72) Inventor: GRASS, Michael, 5656 AE Eindhoven (NL); KOEHLER, Thomas, 5656 AE Eindhoven (NL)
(74) Representative: de Haan, Poul Erik

(57) **Abstract**

The invention relates to a data processor (1) for processing 3D radiographic imaging data. The processor comprises an input (2) for receiving the imaging data and for providing a motion signal indicative of a motion of the imaged subject. The processor comprises an image segmenter (4) for segmenting a body part of interest in a first image included in or derived from the imaging data, and a gating function calculator (5) for calculating temporal gating functions for voxels that belong to the body part taking the motion signal into account. The processor comprises an image reconstructor (3) for reconstructing the imaging data into reconstructed three-dimensional images, by taking the temporal gating functions into account such as to associate each of the reconstructed three-dimensional images with a corresponding phase of the motion.

## Description

### FIELD OF THE INVENTION

The present invention relates to the field of dynamic three-dimensional radiographic imaging, e.g. computed tomography (CT) imaging, using a signal representative of a motion of the imaged subject. More specifically it relates to a data processor, systems comprising such processor, and a method for processing data.

### BACKGROUND OF THE INVENTION

It is known in the art that radiographic projection data of a subject can be acquired while the subject is at least locally in motion, such as due to breathing. Such motion during the imaging can cause image artifacts, such as blurring. Even when a dynamic assessment of the moving body parts, such as the lungs, is not required, the motion may still affect the image quality if the time over which the projection data is acquired is not sufficiently short relative to the length of the periodic motion. However, if the motion is accounted for in a suitable manner, as known in the art, dynamic 3D imaging can be achieved by reconstructing a temporal series of images, e.g. of the breathing subject, by, for example, correlating time indices of the projection data acquisitions with a plurality of time windows during a substantially periodic motion and aggregating the projection data corresponding to each time window to generate a reconstructed image representative of that time window.

For example, in respiratory-gated CT imaging, e.g. breathing gated helical CT scanning, a patient may be scanned, followed by a retrospective sorting of the acquired image data, e.g. of the acquired projection images, into respiratory states. These respiratory states may be defined as a function of breathing amplitude, for example by binning the breathing amplitude. In amplitude binning, the two respiratory signal extremes, i.e. the end-inhale and the end-exhale state, may be detected, and the breathing amplitude range defined by these extremes may be partitioned into bins. The data may thus be divided into, for example, ten bins according to breathing amplitude signal thresholds such that these bins are representative of ten corresponding respiratory states, e.g. ranging from an end-exhale over an end-inhale to an end-exhale position.

In respiratory gated CT scanning of, for example, the lungs, temporal gating windows may be calculated based on a breathing signal. The breathing signal may be measured, for example by a breathing belt, contemporaneous with the CT data acquisition. It is known in the art to use a single set of temporal gating windows, when reconstructing breathing gated images, for all voxels inside the field-of-view, e.g. in the field-of-view of the desired reconstruction.

For example, US 2016/113614 discloses a method for computed tomography data-based cycle estimation and four-dimensional reconstruction. A gated reconstruction is derived from CT data that was acquired without gating by using an added artificial trigger. The resulting images for different slices are used to determine local or slice variations over time. The local variations over time for the various slices are combined to create a respiratory cycle signal. This respiratory cycle signal is used to bin the images for different phases, allowing four-dimensional CT reconstruction.

For a large field-of-view, i.e. in which at least some voxels in the reconstructed image(s) can be at large distance from the main longitudinal scanner axis. E.g. the axis of rotation of the gantry, the gating windows may need to be sufficiently large. Particularly, for achieving a sufficient image quality, the size of the gating window may need to increase with increasing voxel distance from the rotation axis. This can be particularly disadvantageous when reconstructing large field-of-view images, e.g. obtained by a big-bore CT scanner, since the large reconstruction field-of-view may imply a low temporal resolution.

However, in a typical application, as known in the art, the purpose of the respiratory gated dynamic CT scan is to determine the motion of a relatively small feature of interest, such as a tumor in the lungs, whereas the motion of a peripheral body parts, such as the rib-cage, may be of no or less importance. Nonetheless, a large field-of-view reconstruction may still be of interest, e.g. to assess the location of the primary feature of interest in relation to its local and more global surroundings.

### SUMMARY OF THE INVENTION

It is an object of embodiments of the present invention to provide a good image quality of reconstructed images having a large field-of-view in dynamic imaging of a moving subject, e.g. due to breathing, while also achieving a good temporal resolution.

The above objective is accomplished by a method and device according to the present invention.

It is an advantage of embodiments of the present invention that good large field-of-view image reconstructions, i.e. in which at least some voxels in the reconstructed image(s) can be at large distance from the main longitudinal scanner axis, can be achieved with a good temporal resolution during a dynamic imaging of a moving body part.

It is an advantage of embodiments of the present invention that good large field-of-view image reconstructions can be achieved with a good temporal resolution during a dynamic imaging of a moving body part using a big-bore computed tomography (CT) system.

It is an advantage of embodiments of the present invention that feature of interest, e.g. a relatively small feature of interest, such as a lung tumor, can be imaged with a good image quality and a good temporal resolution while also visualizing the feature in a large field-of-view.

It is an advantage of embodiments of the present invention that respiratory gated CT scans obtained from a big-bore CT scanner can be reconstructed with an acceptable temporal resolution and a good image quality.

It is an advantage of embodiments of the present invention that means and methods are provided that are particularly suitable for diagnostic dynamic imaging, e.g. diagnostic 4D imaging, of the lungs and airways.

In a first aspect, the present invention relates to a data processor for processing three-dimensional radiographic imaging data. The data processor comprises an input for receiving the three-dimensional radiographic imaging data and for providing a motion signal indicative of a motion of an imaged subject during the imaging. The data processor comprises an image segmenter for segmenting a body part of interest in a first image included in or derived from the three-dimensional radiographic imaging data. The data processor comprises a gating function calculator for calculating a first set of temporal gating functions for voxels that belong to the body part of interest, as determined by the segmenter, taking the motion signal into account. The data processor comprises an image reconstructor for reconstructing the three-dimensional radiographic imaging data into a plurality of second reconstructed three-dimensional images, by taking the first set of temporal gating functions into account such as to associate each of the second reconstructed three-dimensional images with a corresponding phase of the motion.

In a data processor in accordance with embodiments of the present invention, the body part of interest may be a lung, the lungs, or a part thereof.

In a data processor in accordance with embodiments of the present invention, the input may be adapted for receiving the three-dimensional radiographic imaging data in the form of helical computed tomography image data.

In a data processor in accordance with embodiments of the present invention, the input may be adapted for receiving the helical computed tomography image data generated by a computed tomography scanner, e.g. a big-bore computed tomography scanner. The computed tomography scanner may comprise a cone-beam computed tomography system, e.g. a flat panel detector cone beam computed tomography system.

In a data processor in accordance with embodiments of the present invention, the motion signal may be a breathing signal indicative of a respiratory motion of the subject.

In a data processor in accordance with embodiments of the present invention, the input may be adapted for receiving the motion signal indicative of the motion of the imaged subject from an external motion tracking device.

In a data processor in accordance with embodiments of the present invention, the input may comprise a motion signal generator for generating the motion signal based on the three-dimensional radiographic imaging data.

In a data processor in accordance with embodiments of the present invention, the image segmenter may be adapted for using an image-based and/or model-based segmentation algorithm to determine the pixels or voxels in the first image that belong to the body part of interest.

In a data processor in accordance with embodiments of the present invention, the image segmenter may be adapted for using a thoracic or visceral cavity model to segment the body part of interest.

In a data processor in accordance with embodiments of the present invention, the image segmenter may be adapted for segmenting the body part of interest including a tolerance margin, e.g. of a predetermined distance around the body part or a predetermined number of voxels around the body part, around the body part of interest.

In a data processor in accordance with embodiments of the present invention, the first image used by the image segmenter may be a scout scan image included in the three-dimensional radiographic imaging data.

In a data processor in accordance with embodiments of the present invention, the image reconstructor may be adapted for reconstructing the three-dimensional radiographic imaging data in a first field-of-view to generate a first reconstructed three-dimensional image, wherein the first image used by the image segmenter is the first reconstructed three-dimensional image.

In a data processor in accordance with embodiments of the present invention, the image reconstructor may be adapted for reconstructing the plurality of second reconstructed three-dimensional images in a second field-of-view that is smaller than the first field-of-view.

In a data processor in accordance with embodiments of the present invention, the image reconstructor may be adapted for generating the first reconstructed three-dimensional image without compensating for or taking into account the motion, e.g. the motion signal.

In a data processor in accordance with embodiments of the present invention, the image reconstructor may be adapted for reconstructing the first reconstructed three-dimensional image at a resolution lower than the resolution of the second reconstructed 3D images, and/or for reconstructing the first reconstructed three-dimensional image from a down-sampled version of the three-dimensional radiographic imaging data.

In a data processor in accordance with embodiments of the present invention, the gating function calculator may be adapted for determining a first plurality of gating windows associated with a first plurality of phases of the motion.

In a data processor in accordance with embodiments of the present invention, the image reconstructor may comprise a raw data grouping unit for grouping the three-dimensional radiographic imaging data of a same phase of the motion in a group based on the first plurality of gating windows, and the image reconstructor may be adapted for reconstructing an image for each group from the three-dimensional radiographic data associated with the respective group to generate the second reconstructed three-dimensional images.

In a data processor in accordance with embodiments of the present invention, the gating function calculator may be adapted for calculating a second set of temporal gating functions for (at least) the voxels that do not belong to the body part of interest as determined by the image segmenter, taking the motion signal into account, and the image reconstructor may be adapted for reconstructing the three-dimensional radiographic imaging data into a plurality of third reconstructed three-dimensional images by taking the second set of temporal gating functions into account.

A data processor in accordance with embodiments of the present invention may comprise an image combiner for merging at least one of the second reconstructed three-dimensional images with the first reconstructed three-dimensional image or with at least one of the third reconstructed three-dimensional images, such that voxels belonging to the segmented body part of interest are taken from the second reconstructed three-dimensional image and voxels that do not belong to the segmented body part of interest are taken from the other image (i.e. the first or third reconstructed three-dimensional image).

In a data processor in accordance with embodiments of the present invention, the image reconstructor may be adapted for masking voxels in the plurality of second reconstructed three-dimensional images that do not belong to the body part of interest by setting these voxels to a predetermined background voxel value.

In a second aspect, the present invention relates to a system comprising a data processor in accordance with embodiments of the first aspect of the present invention and a respiration phase tracking device for providing the motion signal to the input.

In a third aspect, the present invention relates to a system comprising a data processor in accordance with embodiments of the first aspect of the present invention and a computed tomography scanner, such as a big-bore computed tomography scanner and/or cone-beam computed tomography scanner, operably connected to the input to provide the three-dimensional radiographic imaging data in the form of helical computed tomography image data. The system may comprise, or may be comprised in, a radiation therapy planning system and/or a radiation therapy system. The system may comprise an on-board imaging system, e.g. in a radiation therapy system or radiation therapy planning system. The system may comprise a flat panel detector cone-beam computed tomography system, e.g. having a centered and/or off-centered detector.

Particular and preferred aspects of the invention are set out in the accompanying independent and dependent claims. Features from the dependent claims may be combined with features of the independent claims and with features of other dependent claims as appropriate and not merely as explicitly set out in the claims.

These and other aspects of the invention will be apparent from and elucidated with reference to the embodiment(s) described hereinafter.

### BRIEF DESCRIPTION OF THE DRAWINGS

FIG 1 shows a data processor in accordance with embodiments of the present invention.
FIG 2 shows a system in accordance with embodiments of the present invention.
FIG 3 shows a method in accordance with embodiments of the present invention.

The drawings are only schematic and are non-limiting. In the drawings, the size of some of the elements may be exaggerated and not drawn on scale for illustrative purposes.

Any reference signs in the claims shall not be construed as limiting the scope.

In the different drawings, the same reference signs refer to the same or analogous elements.

### DETAILED DESCRIPTION OF ILLUSTRATIVE EMBODIMENTS

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.

Where in present description is referred to three-dimensional (3D) radiographic imaging, reference is made to imaging modalities in which ionizing radiation is applied to discern an internal structure of a subject being imaged, such computed tomography (CT) imaging. Particularly, the subject may be imaged from a plurality of angles such as to enable a 3D reconstruction of the imaged subject.

Where in present description is referred to dynamic 3D radiographic imaging, reference is made to radiographic imaging to obtain a sequence of reconstructed image volumes representative of different moments in time. For example, the 'dynamic radiographic imaging' may refer to the imaging of changes that occur in the imaged subject on a time scale that is generally less than 30 minutes, e.g. less than 10 minutes, e.g. has a substantial frequency component in the 0.1 Hz to 10 Hz range. Particularly, the 'dynamic radiographic imaging' may refer to the imaging of changes in the imaged subject that correlate, e.g. strongly correlate, to a motion, e.g. the respiratory motion, of the subject.

In a first aspect, the present invention relates to a data processor for processing three-dimensional radiographic imaging data. The data processor comprises an input for receiving the three-dimensional radiographic imaging data and for providing a motion signal indicative of a motion of an imaged subject during the imaging. The data processor comprises an image segmenter for segmenting a body part of interest in a first image included in or derived from the three-dimensional radiographic imaging data. The data processor comprises a gating function calculator for calculating a first set of temporal gating functions for voxels that belong to the body part of interest, as determined by the segmenter, taking the motion signal into account. The data processor comprises an image reconstructor for reconstructing the three-dimensional radiographic imaging data into a plurality of second reconstructed three-dimensional images, by taking the first set of temporal gating functions into account such as to associate each of the second reconstructed three-dimensional images with a corresponding phase of the motion.

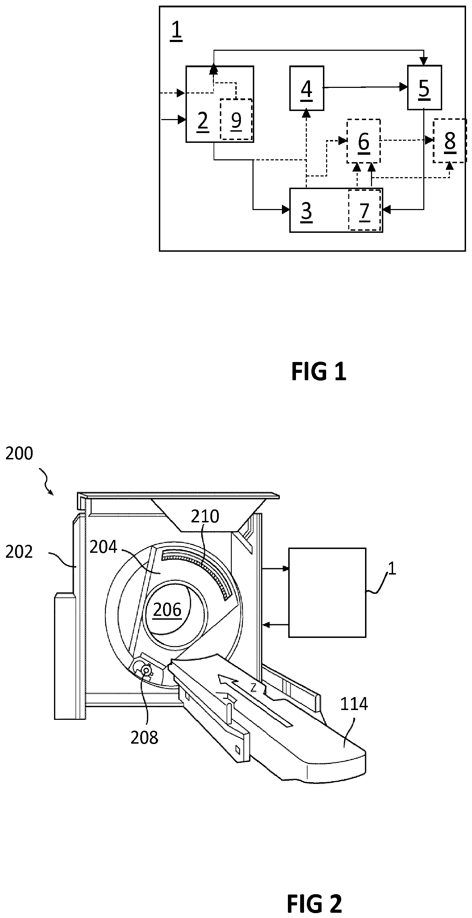

FIG 1 shows a data processor 1, in accordance with embodiments of the present invention, for processing three-dimensional radiographic imaging data, e.g. for processing tomographic diagnostic imaging data, such as computed tomography (CT) diagnostic imaging data. The data processor may use an input signal indicative of a motion of the imaged subject to generate a plurality of reconstructed images representative of different phases of the motion, e.g. different breathing cycle phases.

The data processor 1 may comprise a computer. For example, the data processor 1 may be integrated in an operator console or workstation associated with a medical imaging system. The data processor may comprise a human readable output device such as a monitor or display and a human interface input device such as a keyboard and mouse. An operator may interact with the data processor in a non-interactive or interactive manner, e.g. using a graphical user interface or otherwise. The data processor may be adapted for processing image data provided by a medical imaging system, e.g. a CT scanning unit.

The data processor 1 comprises an input 2 for receiving the three-dimensional radiographic imaging data, such as helical CT image data, e.g. a helical CT scan of the thorax. The radiographic imaging data may be 'raw' radiographic imaging data, e.g. corresponding to radiographic projections through the images subject from a plurality of different angles and acquired at a plurality of different times. For example, the radiographic imaging data may comprise sinogram data.

The radiographic imaging data may be obtained from a CT scanner having a predetermined full field-of-view, e.g. defined by the area circumscribed by a rotating gantry of the CT scanner or, typically, a maximal part of this area that is usable for image reconstruction.

For example, the 3D radiographic imaging data may be obtained from a big-bore CT scanner. A big-bore, or alternatively wide-bore, CT scanner may comprise a computed tomography system, as known in the art, that has a gantry opening of at least 75 cm, e.g. in the range of 75 cm to 125 cm, e.g. in the range of 80 cm to 90 cm, e.g. 85 cm. The full field-of-view may refer to a maximal usable area of the gantry opening for which useful image data can be reconstructed, and may be referred to as an 'extended field-of-view' for the big-bore CT scanner. For example, the gantry opening may measure 80 cm in diameter, and may be configured for reconstructing, using extended field-of-view techniques, a region measuring 65 cm. In another example, a gantry opening of an exemplary CT system may be 85 cm, and may allow an extended field-of-view reconstruction of 60 cm. In another example, the focus to detector distance, corresponding to the gantry opening, may measure 82 cm, and an extended field-of-view may be reconstructed up to 82 cm, e.g. compared to, for example, 50 cm for a standard field-of-view reconstruction.

The input 2 is also adapted for providing a motion signal indicative of a motion of the imaged subject during the acquisition of the 3D radiographic imaging data.

For example, the input 2 may be adapted for receiving the motion signal indicative of the motion of the imaged subject, e.g. from an external device, and/or the input 2 may comprise a motion signal generator 9 for generating the motion signal based on an analysis of the 3D radiographic imaging data, e.g. without relying on an additional input from an external device.

The input 2 may comprise an input port such as a data communication network connection or a dedicated device link, such as a data bus connection, for connecting the data processor to the external device, e.g. a respiration phase tracking device, and/or to a system for providing the three-dimensional radiographic imaging data, e.g. to a CT scanner.

The motion signal may be a breathing signal indicative of a respiratory motion of the subject. The motion signal may be a breathing signal obtained, e.g. by the input, from a respiration phase tracking device. For example, the breathing signal may be obtained from a device for measuring perspiration, e.g. a spirometer, or another device for generating a signal indicative of the breathing motion, e.g. a breathing belt, as known in the art. In another example, the motion signal may be a breathing signal obtained from a vital signs monitoring system. While such vital signs monitoring system may comprise a sensor for directly or indirectly determining the breathing motion, it is also known in the art that a vital signs monitoring system may use an analysis of video images to infer the breathing motion, e.g. may implement a video-based respiration monitoring method for detecting a respiratory region of interest and breathing signal using a camera.

The motion signal may also be derived from the 3D radiographic imaging data, e.g. by inferring the breathing motion from the image content and generating a signal representative of the breathing. Such signal may be received by the input from an external source, e.g. a device for analyzing the image data, e.g. the (raw) projection image data and/or reconstructed images from the projection image data, to infer the breathing motion. However, embodiments of the present invention are not limited thereto. For example, the input may comprise a motion analyzer 3 to generate the signal representative of the respiratory motion from the acquired 3D radiographic imaging data. For example, a method or device as disclosed in US 2016/113614 may be performed, respectively included, for computed tomography data-based cycle estimation, i.e. to determine the motion signal from analyzing the radiographic imaging data.

The data processor comprises an image reconstructor 3.

The image reconstructor 3 may be adapted for reconstructing the three-dimensional radiographic imaging data in a first field-of-view, e.g. a full field-of-view, e.g. the full field-of-view of the CT scanner, thus generating a first reconstructed 3D image. For example, the 3D radiographic imaging data may be reconstructed without restricting a reconstruction field-of-view to a field-of-view that is substantially smaller than the available field-of-view, e.g. the extended field-of-view of a big-bore CT, based on the 3D radiographic imaging data. The 3D radiographic imaging data may be reconstructed, to generate the first reconstructed 3D image, without compensating for or taking into account the motion, e.g. without applying a respiratory gating technique. Thus, the first reconstructed 3D image may be representative of an averaged 3D image, e.g. motion-blurred, over the entire time during which the subject was imaged.

The image reconstructor 3 may be adapted for reconstructing the first reconstructed 3D image at a low resolution, e.g. lower than the second reconstructed 3D images referred to hereinbelow.

The image reconstructor 3 may be adapted for reconstructing the first reconstructed 3D image from a down-sampled version of the three-dimensional radiographic imaging data.

Thus, advantageously, the first reconstructed 3D image can be generated in a fast, simple and/or computationally efficient manner.

The data processor 1 comprises an image segmenter 4, e.g. a segmentation unit, for segmenting a body part of interest, e.g. a part of a lung such as a lobe, a lung or the lungs, in a first image included in or derived from the three-dimensional radiographic imaging data, e.g. in the first reconstructed 3D image referred to hereinabove.

For example, the segmenter 4 may use an image-based and/or model-based segmentation algorithm to determine the pixels or voxels in the first image that belong to the body part of interest. The segmentation processor may segment the body part of interest including a tolerance margin around the body part of interest, e.g. may determine the voxels in the first reconstructed 3D image that belong to the body part or to the tolerance margin around the body part, e.g. the lungs with a safety margin to avoid excluding a part of the lungs in the segmented volume by inaccuracies of the applied segmentation algorithm and/or due to motion blurring in the first reconstructed image.

For example, the segmenter 4 may use a visceral cavity model to detect the body part of interest. A visceral cavity model, as known in the art, may model the inner organs in the visceral cavity, e.g. including the liver. For example, the body part of interest may be the lungs, the liver, the intestines, the stomach, or, generally, a part of interest in the abdominal and/or thoracic cavity.

For example, the segmenter 4 may segment the ribs, e.g. using an image-based and/or model-based segmentation algorithm.

In embodiments of the present invention, the first image used by the segmenter 4 may be another image included in or derived from the three-dimensional radiographic imaging data, i.e. is not necessarily the first reconstructed 3D image described hereinabove. In some embodiments, the first image may be a two-dimensional image. For example, the first image may be scout scan, e.g. a CT scout scan. For example, a scout scan may be a projection radiograph generated by maintaining the CT gantry in a fixed position while the subject is translated through the X-ray beam. Even though the segmentation of a (2D) scout scan does not provide accurate 3D information about the body part of interest, the segmenter may deduce an (e.g. rough) estimate of the voxels in the scanned 3D volume that are likely to belong to the body part of interest, e.g. by fitting a 3D model to the 2D contour of the segmentation in the 2D model.

The data processor 1 comprises a gating function calculator 5 for calculating a first set of temporal gating functions for all voxels that belong to the body part of interest (e.g. to the body part including a tolerance margin) as determined by the segmenter, taking the motion signal into account. Furthermore, the gating function calculator 5 may take scanner acquisition parameters into account to determine the first set of temporal gating functions.

The gating function calculator 5 may determine a first plurality of gating windows, e.g. time segments, associated with a first plurality of phases of the motion, e.g. such that the breathing cycle is partitioned into a number of discrete segments. For example, the motion may be cyclic, or at least repetitive, and the gating function calculator may determine for each instant in time, over the time frame in which the three-dimensional radiographic imaging data was acquired, a corresponding phase of the motion.

The image reconstructor 3 is adapted for reconstructing the three-dimensional radiographic imaging data into a plurality of second reconstructed 3D images. The term "second" does not necessarily imply that the "first" reconstructed 3D image was also reconstructed, in operation of the device, by the same embodiment of the present invention. The second reconstructed 3D images may have a smaller field-of-view than the first field-of-view. The second reconstructed 3D images are reconstructed by taking the first set of temporal gating functions into account such as to associate each of the second reconstructed 3D images with a corresponding phase of the motion.

For example, the image reconstructor 3 may comprise a raw data grouping unit 7 for grouping the 3D radiographic imaging data, e.g. the raw data, of a same phase of the motion in a group based on the first plurality of gating windows, and the image reconstructor may be adapted for reconstructing an image for each group from the 3D radiographic data associated with the respective group.

For example, the image reconstructor 3 may perform a gated reconstruction, e.g. a breathing-gated, reconstruction for all voxels which belong to the body part of interest, e.g. the lung, taking the motion signal, e.g. a breathing signal, into account.

The gating function calculator 5 may be adapted for calculating a second set of temporal gating functions for all voxels that do not belong to the body part of interest (e.g. do not belong to the body part including the tolerance margin) as determined by the segmenter, taking the motion signal into account. Furthermore, the gating function calculator 5 may take scanner acquisition parameters into account to determine the second set of temporal gating functions.

The gating function calculator 5 may also be adapted for determining a second plurality of gating windows, e.g. time segments, associated with a second plurality of phases of the motion, e.g. such that the breathing cycle is partitioned into a number of discrete segments.

It is to be noted that, while the first and second set of temporal gating functions and/or the first and second plurality of gating windows relate to the same motion, this motion is not necessarily, e.g. typically not, partitioned by the first and second set of temporal gating functions into the same plurality of phases. For example, at least some of the voxels considered for the second set of temporal gating functions may be more peripheral from the scanner axis than any of the voxels considered for the first set of temporal gating functions. Therefore, the raw data available for reconstructing these more peripheral voxels may be more sparse, e.g. less densely sampled, and/or may be subject to more noise relative to the acquired signal. The gating function calculator may take an image quality metric, e.g. a signal-to-noise ratio but not necessarily limited thereto, into account for determining the number of phases into which the motion is partitioned for the first plurality of gating windows, and/or for determining the number of phases into which the motion is partitioned for the second plurality of gating windows.

The image reconstructor 3 may also be adapted for reconstructing the three-dimensional radiographic imaging data into a plurality of third reconstructed 3D images. The third reconstructed 3D images may have the same field-of-view as the first field-of-view, or, at least, a larger field-of-view than the second reconstructed 3D images. The third reconstructed 3D images are reconstructed by taking the second set of temporal gating functions into account such as to associate each of the third reconstructed 3D images with a corresponding phase of the motion.

The plurality of third reconstructed 3D images may consist of less images than the plurality of second reconstructed 3D images, e.g. due to a lower temporal resolution that can be achieved when reconstructing more peripheral voxels (relative to the scanner axis) outside the body part of interest.

The data processor 1 may comprise an image combiner 6 for merging the first reconstructed 3D image with at least one of the second reconstructed 3D images, e.g. such that voxels that belong to the segmented body part of interest are taken from the second reconstructed image and voxels that do not belong to the segmented body part of interest are taken from the first reconstructed image. For example, the image combiner 6 may generate a merged image for each of the second reconstructed 3D images in combination with the first reconstructed 3D image.

Alternatively, or additionally, the image combiner 6 may be adapted for merging at least one of the second reconstructed 3D images with at least one of the third reconstructed 3D images, e.g. such that voxels that belong to the segmented body part of interest are taken from the second reconstructed image and voxels that do not belong to the segmented body part of interest are taken from the third reconstructed image. For example, the image combiner 6 may generate a merged image for each of the second reconstructed 3D images in combination with the third reconstructed 3D image that corresponds with the motion phase for which the second reconstructed 3D image was reconstructed, that corresponds the best, such having as the largest overlap of the motion phase corresponding to the second reconstructed 3D image and the motion phase corresponding to the third reconstructed 3D image.

However, instead of combining images, in embodiments in accordance with the present invention, voxels that lie outside the segmented body part of interest may also be masked, e.g. set to predetermined background voxel value, in the plurality of second reconstructed 3D images.

The data processor 1 may also comprise an output 8 for outputting the plurality of second reconstructed three-dimensional images, the plurality of masked second reconstructed three-dimensional images and/or the merged image(s) generated by the image combiner 6.

In a second aspect, the present invention relates to a system comprising a data processor in accordance with embodiments of the first aspect of the present invention and a respiration phase tracking device for providing the motion signal to the input.

For example, the respiration phase tracking device may comprise a real-time video analysis system for estimating a value indicative of a present lung volume in the respiration cycle of a subject being observed, such as, for example, a video-based respiratory gating system.

However, the respiration phase tracking device may also comprise an alternative system for estimating a value indicative of a present lung volume in the respiration cycle of a subject being observed, e.g. based on a spirometer measurement.

The respiration phase tracking device may comprise a breathing belt. For example, the respiration phase tracking device may comprise a sensor for transducing changes in the length of the breathing, as indicative of the circumference around the chest of the subject, in operation of the device, and thus as indicative of the breathing phase. For example, such sensor may comprise a piezo-electric element for responding, e.g. linearly, to changes in length of the belt.

Furthermore, it shall be clear to the person skilled in the art that the respiration phase tracking device may be co-integrated with the data processor, e.g. such that processing for estimating a value indicative of a present lung volume in the respiration cycle of a subject being observed may be carried out by the processor of the radiation emission controller.

In a third aspect, the present invention relates to a system comprising a data processor in accordance with embodiments of the first aspect of the present invention and a computed tomography scanner, such as a big-bore computed tomography scanner, operably connected to the input to provide the three-dimensional radiographic imaging data in the form of helical computed tomography image data.

A system in accordance with embodiments of the third aspect of the present invention may also comprise a respiration phase tracking device as described hereinabove for embodiments of the second aspect of the present invention.

For example, such computed tomography scanner 200 is illustrated in FIG 2. In a system in accordance with embodiments of the present invention, a CT scanning unit 200 may be adapted for performing multiple axial scans and/or a helical scan of a subject, e.g. of the thorax region of a patient.

For example, the CT scanning unit, e.g. the computed tomography scanner, may comprise a stationary gantry 202 and a rotating gantry 204, which may be rotatably supported by the stationary gantry 202. The rotating gantry 204 may rotate, about a longitudinal axis, around an examination region 206 for containing the subject to be imaged when acquiring projection data. The CT scanning unit may comprise a subject support 114, such as a couch, to support the subject in the examination region 206.

The CT scanning unit may comprise an x-ray tube 208, which may be supported by and configured to rotate with the rotating gantry 204. This radiation source may include an anode and a cathode. A source voltage applied across the anode and the cathode may accelerate electrons from the cathode to the anode. The electron flow may provide a current flow from the cathode to the anode, such as to produce radiation for traversing the examination region 206.

The CT scanning unit may comprise a detector array 210. This detector array may subtend an angular arc opposite the examination region 206 relative to the radiation source 208. The detector array may include a one or two dimensional array of pixels, e.g. detector pixels. The detector array may be adapted for detecting radiation traversing the examination region and for generating a signal indicative of an energy thereof.

The CT scanning unit may be a big-bore (or wide-bore or large-bore) CT scanning unit, e.g. in which the distance between the focus of the x-ray tube 208 and the detector array 210 is at least 75 cm, e.g. in the range of 75 cm to 125 cm, e.g. in the range of 80 cm to 90 cm, e.g. 85 cm. For example, for radiation therapy planning, the subject may need to be imaged in a position in which the subject cannot enter a conventional bore opening, e.g. of 70 cm or less. Therefore, it is a particular advantage of a big-bore CT scanner that it can be used for planning radiation therapy treatments due to a larger-than-conventional bore size.

The CT scanning unit may be configured to perform a plurality of image acquisitions, e.g. to acquire a plurality of projection images, e.g. a plurality of CT projections. This plurality of image acquisitions may be performed in accordance with a predetermined or configurable imaging sequence, e.g. defined in an acquisition schedule. For example, such acquisition schedule may comprise a frequency setting for generating a plurality of uniformly distributed imaging pulses, in which an image is to be acquired for each imaging pulse.

In a fourth aspect, the present invention relates to a method for processing three-dimensional radiographic imaging data. The method comprises receiving the three-dimensional radiographic imaging data. The method comprises providing a motion signal indicative of a motion of an imaged subject during the imaging. The method comprises segmenting a body part of interest in a first image included in or derived from the three-dimensional radiographic imaging data. The method comprises calculating a first set of temporal gating functions for voxels that belong to the body part of interest, as determined by the segmentation, taking the motion signal into account. The method comprises reconstructing the three-dimensional radiographic imaging data into a plurality of second reconstructed three-dimensional images, by taking the first set of temporal gating functions into account such as to associate each of the second reconstructed three-dimensional images with a corresponding phase of the motion.

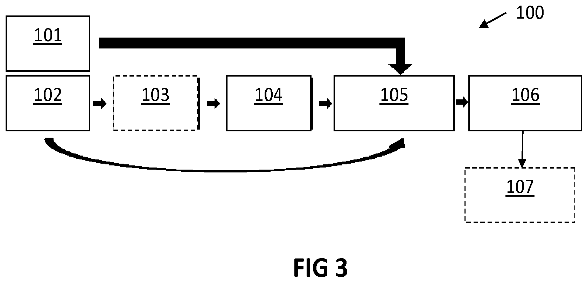

Referring to FIG 3, a method 100 for processing three-dimensional radiographic imaging data in accordance with embodiments of the present invention is shown.

The method comprises receiving 102 the three-dimensional radiographic imaging data, e.g. big-bore CT projection data. For example, the three-dimensional radiographic imaging data may be obtained in the form of helical computed tomography image data. The step of receiving 102 may comprise receiving the helical computed tomography image data generated by a big-bore computed tomography scanner (or may comprise generating the data by a big-bore CT scanner).

The method comprises providing 101 a motion signal indicative of a motion of an imaged subject during the imaging. The motion signal may be a breathing signal indicative of a respiratory motion of the subject. The step of providing 101 the motion signal may comprise receiving the motion signal indicative of the motion of the imaged subject from an external motion tracking device, e.g. a breathing belt or another device for determining a breathing motion. The step of providing 101 the motion signal may comprise generating the motion signal based on the three-dimensional radiographic imaging data.

The method may comprise reconstructing 103 the three-dimensional radiographic imaging data in a first field-of-view to generate a first reconstructed three-dimensional image.

The step of reconstructing 103 may comprise generating the first reconstructed three-dimensional image without compensating for or taking into account the motion, e.g. the motion signal.

Reconstructing 103 the first reconstructed three-dimensional image may comprise reconstructing the first reconstructed 3D image at a resolution lower than the resolution of the second reconstructed 3D images, and/or reconstructing the first reconstructed three-dimensional image from a down-sampled version, e.g. sparsely sampled, of the three-dimensional radiographic imaging data.

The method comprises segmenting 104 a body part of interest, e.g. a lung, the lungs or a part thereof, in a first image included in or derived from the three-dimensional radiographic imaging data.

The step of segmenting 104 may use an image-based and/or model-based segmentation algorithm to determine the pixels or voxels in the first image that belong to the body part of interest. The step of segmenting may use a thoracic or visceral cavity model to segment the body part of interest. The step of segmenting may comprise segmenting the body part of interest including a tolerance margin, e.g. of a predetermined distance around the body part or a predetermined number of voxels around the body part, around the body part of interest.

The first image used in the segmentation 104 may be a scout scan image included in the three-dimensional radiographic imaging data.

The first image used by the step of segmenting 104 may be the first reconstructed three-dimensional image reconstructed in the step of reconstructing 103.

The method comprises calculating 105 a first set of temporal gating functions for voxels that belong to the body part of interest, as determined by the segmentation, taking the motion signal into account. Calculating 105 the first set of temporal gating functions may comprise determining a first plurality of gating windows associated with a first plurality of phases of the motion.

The method comprises reconstructing 106 the three-dimensional radiographic imaging data into a plurality of second reconstructed three-dimensional images, by taking the first set of temporal gating functions into account such as to associate each of the second reconstructed three-dimensional images with a corresponding phase of the motion. For example, the step of reconstructing 106 may comprise a breathing-gated reconstruction in a field-of-view encompassing the body part of interest.

In the step of reconstructing 106, the plurality of second reconstructed three-dimensional images may be reconstructed in a second field-of-view that is smaller than the first field-of-view used in the step of reconstructing 103.

The step of reconstructing 106 may comprise grouping the three-dimensional radiographic imaging data of a same phase of the motion in a group based on the first plurality of gating windows, and reconstructing an image for each group from the three-dimensional radiographic data associated with the respective group to generate the second reconstructed three-dimensional images.

The method may also comprise calculating a second set of temporal gating functions for (at least) the voxels that do not belong to the body part of interest as determined by the image segmenter, taking the motion signal into account, and reconstructing the three-dimensional radiographic imaging data into a plurality of third reconstructed three-dimensional images by taking the second set of temporal gating functions into account.

The method may also comprise combining 107, e.g. merging, at least one of the second reconstructed three-dimensional images with the first reconstructed three-dimensional image or with at least one of the third reconstructed three-dimensional images, such that voxels belonging to the segmented body part of interest are taken from the second reconstructed three-dimensional image and voxels that do not belong to the segmented body part of interest are taken from the other image (i.e. the first or third reconstructed three-dimensional image).

The method may also comprise masking voxels in the plurality of second reconstructed three-dimensional images that do not belong to the body part of interest by setting these voxels to a predetermined background voxel value.

## Claims

1. A data processor (1) for processing three-dimensional radiographic imaging data, the data processor comprising:
an input (2) for receiving said three-dimensional radiographic imaging data and for providing a motion signal indicative of a motion of an imaged subject during said imaging;
an image segmenter (4) for segmenting a body part of interest in a first image included in or derived from the three-dimensional radiographic imaging data;
a gating function calculator (5) for calculating a first set of temporal gating functions for voxels that belong to said body part of interest, as determined by said segmenter, taking the motion signal into account, and
an image reconstructor (3) for reconstructing said three-dimensional radiographic imaging data into a plurality of second reconstructed three-dimensional images, by taking said first set of temporal gating functions into account such as to associate each of the second reconstructed three-dimensional images with a corresponding phase of said motion.

2. The data processor of any of the previous claims, wherein said motion signal is a breathing signal indicative of a respiratory motion of said subject.

3. The data processor of any of the previous claims, wherein said input (2) is adapted for receiving said motion signal indicative of the motion of the imaged subject from an external motion tracking device or wherein said input (2) comprises a motion signal generator (9) for generating the motion signal based on said three-dimensional radiographic imaging data.

4. The data processor of any of the previous claims, wherein said image segmenter (4) is adapted for using a thoracic cavity or visceral cavity model to segment said body part of interest.

5. The data processor of any of the previous claims, wherein said image segmenter (4) is adapted for segmenting said body part of interest including a tolerance margin around said body part of interest.

6. The data processor of any of the previous claims, wherein said first image used by said image segmenter (4) is a scout scan image included in said three-dimensional radiographic imaging data.

7. The data processor of any of claims 1 to 5, wherein said image reconstructor (3) is adapted for reconstructing the three-dimensional radiographic imaging data in a first field-of-view to generate a first reconstructed three-dimensional image without compensating for or taking into account said motion, and wherein said first image used by said image segmenter (4) is said first reconstructed three-dimensional image.

8. The data processor of claim 7, wherein said image reconstructor (3) is adapted for reconstructing the plurality of second reconstructed three-dimensional images in a second field-of-view that is smaller than said first field-of-view.

9. The data processor of claim 7 or claim 8, wherein said image reconstructor (3) is adapted for reconstructing said first reconstructed three-dimensional image at a resolution lower than the resolution of said second reconstructed 3D images, and/or for reconstructing said first reconstructed three-dimensional image from a down-sampling of the three-dimensional radiographic imaging data.

10. The data processor of any of the previous claims, wherein said gating function calculator (5) is adapted for determining a first plurality of gating windows associated with a first plurality of phases of said motion, wherein said image reconstructor (3) comprises a raw data grouping unit (7) for grouping said three-dimensional radiographic imaging data of a same phase of said first plurality of phases in a group based on said first plurality of gating windows, and wherein said image reconstructor is adapted for reconstructing an image for each group from the three-dimensional radiographic data associated with the respective group to generate said plurality of second reconstructed three-dimensional images.

11. The data processor of any of the previous claims, wherein said gating function calculator (5) is adapted for calculating a second set of temporal gating functions for the voxels that do not belong to said body part of interest as determined by said image segmenter (4), taking said motion signal into account, and wherein said image reconstructor (3) is adapted for reconstructing said three-dimensional radiographic imaging data into a plurality of third reconstructed three-dimensional images by taking said second set of temporal gating functions into account.

12. The data processor of claim 7 or claim 11, comprising an image combiner (6) for merging at least one of said second reconstructed three-dimensional images with said first reconstructed three-dimensional image or with at least one of said third reconstructed three-dimensional images, such that voxels belonging to said segmented body part of interest are taken from the second reconstructed three-dimensional image and voxels that do not belong to said segmented body part of interest are taken from the other image.

13. A system comprising a data processor in accordance with any of the previous claims and a respiration phase tracking device for providing said motion signal to said input (2).

14. A system (200) comprising a data processor in accordance with any of the claims 1 to 12 and a big-bore computed tomography scanner operably connected to said input (2) to provide said three-dimensional radiographic imaging data in the form of helical computed tomography image data.

15. Method (100) for processing three-dimensional radiographic imaging data, the method comprising:
receiving (102) the three-dimensional radiographic imaging data;
providing (101) a motion signal indicative of a motion of an imaged subject during the imaging;
segmenting (104) a body part of interest in a first image included in or derived from the three-dimensional radiographic imaging data;
calculating (105) a first set of temporal gating functions for voxels that belong to the body part of interest, as determined by said segmentation, taking the motion signal into account, and
reconstructing (106) the three-dimensional radiographic imaging data into a plurality of second reconstructed three-dimensional images, by taking the first set of temporal gating functions into account such as to associate each of the second reconstructed three-dimensional images with a corresponding phase of the motion.
